(19) **Europäisches Patentamt**
**European Patent Office**
**Office européen des brevets**

(11) **EP 4 518 752 B1**

(12) **EUROPEAN PATENT SPECIFICATION**

(45) Date of publication and mention
of the grant of the patent:
**29.10.2025  Bulletin 2025/44**

(21) Application number: **23725364.6**

(22) Date of filing: **05.05.2023**

(51) International Patent Classification (IPC):
*A61B 5/11* [(2006.01)]      *A61B 5/00* [(2006.01)]

(52) Cooperative Patent Classification (CPC):
**A61B 5/11; A61B 5/6829;** A61B 2560/0443

(86) International application number:
**PCT/EP2023/062032**

(87) International publication number:
**WO 2023/214061 (09.11.2023 Gazette 2023/45)**

(54) **EVALUATION OF THE FOREFOOT RELATIVE MOBILITY**

BEURTEILUNG DER RELATIVEN BEWEGLICHKEIT DES VORDERFUSSES

ÉVALUATION DE LA MOBILITÉ RELATIVE DE L'AVANT-PIED

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **06.05.2022  EP 22172071**

(43) Date of publication of application:
**12.03.2025  Bulletin 2025/11**

(73) Proprietor: **FONDATION FORTE**
**1206 Geneva (CH)**

(72) Inventors:
• **PRAZ, Quentin**
**01280 Prévessin-Moëns (FR)**
• **PASSERAUB, Philippe**
**Provo, UT 84604 (US)**
• **SCHOINAS, Spyridon**
**1206 Geneva (CH)**
• **ASSAL, Mathieu**
**1206 Geneva (CH)**
• **ACKER, Antoine**
**1005 Lausanne (CH)**
• **RAYMOND, Nils**
**1206 Geneva (CH)**

(74) Representative: **KATZAROV S.A.**
**Geneva Business Center**
**12 Avenue des Morgines**
**1213 Petit-Lancy (CH)**

(56) References cited:
**WO-A1-2017/171660**

• **GLASOE WARD MYLO ET AL: "Measuring first ray mobility with a new device", ARCHIVES OF PHYSICAL MEDICINE AND REHABILITATION, vol. 80, no. 1, 1 January 1999 (1999-01-01), AMSTERDAM, NL, pages 122 - 124, XP055813238, ISSN: 0003-9993, DOI: 10.1016/ S0003-9993(99)90320-9**

EP 4 518 752 B1

# Description

## Technical Field

The present disclosure relates to a device and a system comprising said device for evaluating the relative mobility of a forefoot of a patient.

## Background of the art

The issue of a non-objective method in the examination of the forefoot relative mobility inspired numerous researchers to develop various methods and instruments. In the 1990s, Klaue and Glasoe were the first to propose and validate a technical solution that was able to quantify the first ray mobility in dorsiflexion. These devices are performing an absolute displacement measurement with the foot in a neutral position while an automatized fixed force or manual unquantified force was only applied under the first metatarsal head. From this, Klaue estimated the threshold of forefoot mobility to be 8 millimeters or more by conducting studies with feet with Hallux Valgus and without. This study revealed a higher mobility for feet with Hallux Valgus.

Based on these pioneered methods and instruments, various other solutions conceived to propose a simpler measuring system that would be closer to the clinical examination. Nevertheless, a "simpler device" does not mean a device relevant for making appropriate therapeutic decisions from the clinical assessment, mainly due to the lack of reliable data generated induced by a great inter-observer variability.

For instance, a plastic instrument has been developed and consists of two millimetric scale pieces placed above the first metatarsal head accompanying the manual examination. It has the advantage of being low-cost and easy to use for daily practice. However the amount of applied force from the practitioner is not standardized, which makes the instability hard to assess. It is also impossible to provide objective monitoring of the pathology.

Other more complex solutions associated the gesture of the practitioner with an optical measurement system that recorded by a camera the angular deformation in dorsiflexion of the first metatarsophalangeal joint while the practitioner applies a force on the first and lesser metatarsal heads to measure a millimetric distance.

The majority of these solutions were used only for medical research purposes due to their numerous disadvantages, such as their lack of reliability, validity or responsiveness, not being practical for daily clinical use and being too complex. In addition, the existing instruments do not allow the assessment of instability reflecting a stance phase, where all metatarsals are loaded by the ground reaction force, which is essential for clinical decision making. None of these studies was able to propose forefoot instability classification correlating the assessment to the clinical decision.

WO 2017/171660 discloses a device that evaluates foot joint mobility by different measurements: the rotational stiffness of the metatarsophalangeal joint and the stiffness of the big toe. This device comprises wearable sensor based on a fiber Bragg grating technology and a handheld instrument measuring applied force to the foot.

Therefore, there is a need to provide an improved approach for measuring relative mobility of the forefoot of a patient to overcome or limit the drawbacks of the existing instruments.

## Summary of the invention

The above problems are solved or at least minimized by the present invention.

The invention concerns a device for evaluating the relative mobility of a forefoot of a patient, the device comprising an actuator, a force sensor, a housing, a transmission element and two output shafts namely a first output shaft and a second output shaft,

the first output shaft being configured for bearing against the first metatarsal head M1 of the forefoot whereas the second output shaft being configured for bearing against the lesser metatarsal heads M2-M5 of the forefoot,

the force sensor is able to measure an axial force exerted by the actuator to the two output shaft bearing to the forefoot

characterized in that the transmission element comprises a pivot plate connecting the housing to the two output shafts, the pivot plate is able to equally distributes the axial force provided by the actuator via the housing, to the first output shaft and to the second output shaft,

and wherein the device comprises a displacement sensor able to measure the angulation of the pivot plate.

In another aspect, the invention concerns a system for evaluating the relative mobility of a forefoot of a patient, the system comprising

a device according to the present invention;

an orthosis configured for maintaining the forefoot of the patient in position suitable for measuring the relative mobility between the first metatarsal head and the lesser metatarsal heads of a forefoot of a patient;

a positioning module configured for reversibly fastening the device to the orthosis.

## Description of the invention

The invention concerns a device for evaluating the relative mobility of a forefoot of a patient, the device comprising an actuator, a force sensor, a housing, a transmission element and two output shafts namely a first output shaft and a second output shaft,

the first output shaft being configured for bearing against the first metatarsal head M1 of the forefoot whereas the second output shaft being configured for bearing against the lesser metatarsal heads M2-M5 of the forefoot,

the force sensor is able to measure an axial force exerted by the actuator to the two output shafts bearing to the forefoot,

characterized in that the transmission element (8) comprises a pivot plate connecting the housing to the two output shafts, the pivot plate is able to equally distributes the axial force provided by the actuator via the housing, to the first output shaft and to the second output shaft,

and wherein the device comprises a displacement sensor able to measure the angulation of the pivot plate.

According to one embodiment, the pivot plate is connected to the housing by a transverse axis and bearings.

According to one characteristic, the first output shaft is mounted on a first end of the pivot plate, and the second output shaft is mounted on the opposed end of the pivot plate.

According to another characteristic, each output shafts comprises an oblong aperture, and each end of the pivot plate comprises a circular hole in which a pin is inserted through the circular hole and the oblong aperture.

According to a complementary characteristic, the device comprises plain bearings interdependent to the housing, wherein the output shafts can freely slide.

According to the previous embodiment, the housing comprises an exterior recess, at its lower ends, wherein the force sensor is embedded.

Advantageously, the present invention allows to evaluate, preferably to quantify, the relative mobility as a function of the force applied between the first metatarsal head and the lesser metatarsal heads under equal force load. This is done by measuring the applied force and the resulting displacement corresponding to said measured applied force.

The functioning of the present invention advantageously replicates or mimics the orthopedic surgeon's gesture during a manual examination of patient forefoot to evaluate the relative stability by manual technics.

In contrast to the other existing methods, the proposed solution is biomechanically representative of a progressive weight-bearing phase of the gait.

Advantageously, the present invention allows to assist practitioners involved in the forefoot treatment, who want to make the most appropriate therapeutic decision for each patient's pathology.

The present invention offers data-driven biomechanical measurements of the forefoot's mobility and ensures objectivity with standardized measurement. It is possible to measure the force applied on the forefoot so that a given load can be reproduced by another practitioner to the one that performs the measurement in the first place. In other words, the present invention rationalizes the measuring of the relevant forefoot parameters (in particular displacement and the force applied) to avoid or at least limit the inter-observer variability. Therefore, the present invention can be considered as an objective method to evaluate the relative mobility of a patient's forefoot.

The present invention allows to personalize the forefoot analysis to each patient by applying the required force to obtain a relevant displacement measurement.

Preferably, the axial force applied by the actuator on the forefoot is exerted along the sagittal axis, more preferably in the dorsal direction.

Preferably, the relative displacement of the first metatarsal head M1 relative to the lesser metatarsal heads M2-M5 is measured along the sagittal axis, more preferably in the dorsal direction.

In a preferred embodiment, the force sensor and the displacement sensor simultaneously measure the force applied and the angulation of the pivot plate. Thus, each measured displacement corresponds to a measured applied force. This allows to associate to a measured applied force a corresponding displacement measurement. Preferably, the measurement of the relative displacement corresponding to an applied force are measured simultaneously, meaning with an interval less than or equal to about 1 ms.

Preferably, the device is configured for evaluating the relative mobility of the patient when the patient is in a standing position and in a lying position and any positions between a standing position and a lying position. In other words, the present invention can be used in multiple configurations, namely with a patient in a standing up position or in a lying position or in another position in between.

The lying position corresponds to the position of a patient examined by a practitioner.

When the device (or system) is used on a patient in a seated position (as an example of a position between a lying position and a standing position, the patient simulates the load on the forefoot of a standing position during evaluation, but with less force applied on said forefoot, meaning a partial weight bearing, for instance about 40% less. For instance, the vertical force of the lower limb is approximately 10% of the total body weight.

When the device (or system) is used on a patient in a standing position, the patient simulates the maximum static load on the forefoot during evaluation.

In a preferred embodiment the actuator is configured for being actuated manually by a user of the device providing the axial force, preferably the actuator comprising a hand to be handled by the user. For instance, the actuator is handled by the practitioner taking the measurements of a forefoot of a patient to evaluate the relative mobility of said forefoot. The force applied on the forefoot is transmitted by the practitioner to the forefoot via the actuator. Advantageously, a manual actuator provides a simple and inexpensive device with a small footprint, notably a low energy consumption and a low $CO_2$ footprint.

Preferably, the actuator is configured for being actuated by driving element commanding the actuation, preferably a motor, to provide the axial force. The present invention can be driven by a motorized element providing the applied force exerted on the forefoot of the patient. This allows reproducible dynamic measurements. It also reduces the influence of the user on the measurements.

In a preferred embodiment the device further comprises a computation unit configured for processing the measurements acquired by the measuring module to evaluate the relative mobility as a function of the measured axial force with respect to the measured relative displacement.

Preferably, the computation unit is embedded in the housing of the device. For instance, the computation unit comprises a processing unit, such as a microcontroller configured for processing the measured displacements and the measured applied forces to evaluate the relative mobility as a function of the measured axial force with respect to the measured relative displacement. In this embodiment, the device is independent, i.e. autonomous, from any other calculation or computation unit to evaluate the relative mobility as a function of the measured axial force with respect to the measured relative displacement.

Preferably, the computation unit is connected to a display unit to display the data processed by the computation unit, in particular the processing unit, for instance to display the relative mobility as a function of the measured axial force with respect to the measured relative displacement.

Alternatively, in another embodiment the computation unit is outside the housing of the device. The measuring module is connected to the computation unit to transfer the data outside the housing of the device. The processing of the measured displacements and the measured applied forces to evaluate the relative mobility as a function of the measured axial force with respect to the measured relative displacement is performed outside the housing.

Advantageously, the computation unit allows

- to calculate the indicators of interest;

- interact with the device, preferably with the measuring module (to determine Fmin and Fmax for example),

- superimpose the measurements of a same series to calculate the variability (standard deviation);

- export the data in appropriate format, for instance csv.

Preferably, the device further comprises a first metatarsal head M1 tip or support configured for being mounted on the first output shaft to be beard against the first metatarsal head M1, and a lesser metatarsal heads M2-M5 tip configured for being mounted on the second output shaft to be beard against the lesser metatarsal heads M2-M5. This allows to change the tips between two patients to comply with a sanitary protocol, for instance to avoid transmitting pathogens from one patient to another.

In a preferred embodiment the first metatarsal head M1 tip and the lesser metatarsal heads tip M2-M5 are reversibly mounted on respectively the first output shaft and the second output shaft, and wherein the first metatarsal head M1 tip and the lesser metatarsal heads tip M2-M5 are both compatible with the first output shaft and the second output shaft.

In other words, the tips are interchangeable: a same tip, namely the first metatarsal head M1 tip or the lesser metatarsal heads tip M2-M5, fits with the first output shaft and also with the second output shaft. When the device is first used with the right forefoot, the first metatarsal head M1 tip is on the first output shaft whereas the lesser metatarsal heads tip M2-M5 is on the second output shaft. Then, the tips are switched when the device is used with the left forefoot: the first metatarsal head M1 tip is now on the second output shaft whereas the lesser metatarsal heads tip M2-M5 is on the first output shaft. One device can be used with both forefeet by switching the tips.

Advantageously, the tips can be easily cleaned, notably the parts in contact with the skin of the patient. The tip can be chosen according to the morphology of each patient (shape of the tips can change while keeping the same principle of fixing with the output shaft).

Preferably, the transmission module is chosen among a gear transmission, a pivot plate pivotable around a pivot point, preferably a pivot plate pivotable around a pivot point. The transmission module can be any module or components assembly configured for equally distributing the axial force to two output shafts or at least two output shafts. Preferably, the transmission module supports the stresses or forces related to the measurements and reduce friction forces with several cylindrical bearings.

Advantageously, when the axial force is exerted on the transmission element and equally distributed to the first output shaft and to the second output shaft, the transmission element is configured for adopting a position or orientation depending the load exerted by the first output shaft and the second output shaft on the transmission element in response to the axial force exerted. In other words, the transmission element is configured to adopt a position or orientation depending on the relative mobility of the forefoot.

In a preferred embodiment, the transmission module comprises a pivot plate pivotable around a pivot point, the actuator being arranged for exerting an axial force on the pivot point of the pivot plate, the first output shaft being mounted on a first end of the pivot plate and the second output shaft being mounted on the opposed end of the pivot plate.

In an embodiment, when the axial force is exerted on the pivot plate and equally distributed to the first output shaft and to the second output shaft, the pivot plate is configured for adopting a position or orientation depending the load exerted by the first output shaft and the second output shaft on the pivot plate in response to the axial force exerted. In other words, the pivot plate is configured to adopt a position or orientation depending on the relative mobility of the forefoot.

For instance, the pivot plate can adopt either a first position or a second position,

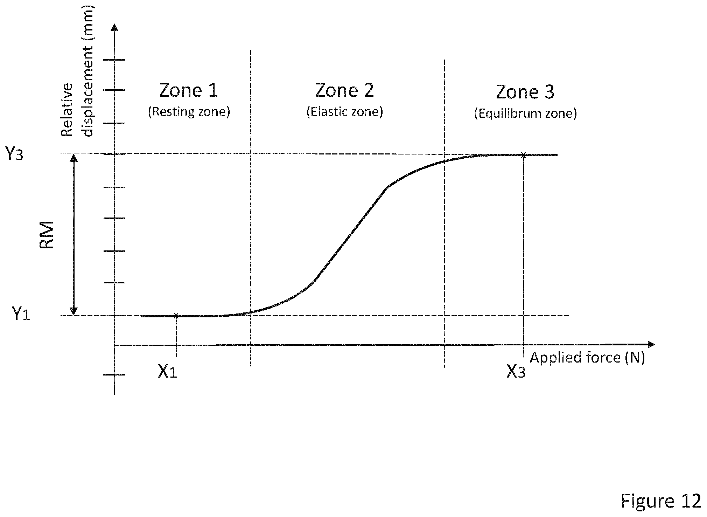

- a first position corresponding to a resting zone (zone 1 in figure 12) where the effort exerted by the first output shaft on the pivot plate is equal to the effort applied by the second output shaft on the pivot plate, and

- a second position corresponding to an equilibrium zone (zone 3 in figure 12) where the effort exerted by the first input shaft on the pivot plate is different from the effort applied by the second input shaft on the pivot plate.

In a preferred embodiment the displacement sensor is chosen among an angular sensor, a magnetic angular sensor, an optical encoder, a mechanical encoder.

In another embodiment the displacement sensor is chosen among a graduated scale, a laser sensor, an electromagnetic sensor for instance a Linear Variable Differential Transformer LVDT, preferably a magnetic angular sensor.

The displacement sensor can be any module or components assembly configured for measuring the relative displacement of the first metatarsal head M1 relative to the lesser metatarsal heads M2-M5 along said sagittal axis when beard respectively against the first output shaft and on the second output shaft.

According to the previous embodiment, the transverse axis comprises at one of its ends a magnet located in front of a magnetic angular sensor.

In a preferred embodiment, the transmission module comprises a pivot plate and the displacement sensor is an angular sensor, said angular sensor being coupled to the pivot point of said pivot plate. The angular sensor is configured for measuring a pivot angle when the pivot plate is pivoting, for instance either clockwise (i.e. positive angle) or counter clockwise (i.e. negative angle). The measured pivot angle is used to compute the measured relative displacement by applying known trigonometry based mathematical formula.

For example, the pivot angle is measured between a first position and a second position of the pivot plate, the first position corresponding to a resting zone and the second position corresponding to an equilibrium zone (see figure 12).

In a preferred embodiment, the displacement sensor is a magnetic angular sensor. Magnetic angular sensors have many advantages such as being non-contact and miniature solution with high angular resolution and high sensitivity, linear characterization and low power consumption.

Preferably, the force sensor is chosen among load cell, compression load cell, spring dynamometer.

In a preferred embodiment, the force sensor is a load cell preferably compression load cell. Such sensors have many advantages such as digital format, good resolution and sensitivity, small footprint, low power consumption and linear characterization.

In an embodiment, the device comprises a feedback module. For instance the feedback module is configured for providing a feedback to the practitioner. Advantageously, the feedback allows the practitioner to evaluate, preferably measure, the force applied on the forefoot, notably to evaluate the increase or decrease of the applied force.

Preferably, the feedback is haptic feedback, visual feedback and/or audible feedback, preferably visual feedback and/or audible feedback.

In a preferred embodiment, the feedback module comprises a visual display to provide visual feedback.

In an embodiment, the feedback module comprises a microphone (or a speaker or a miniature speaker) to emit a sound signal.

Preferably, the feedback given is adaptable to the force applied.

In an embodiment, the present invention, preferably the system, comprises a graduated scale configured for calibrating the distribution of the force between the first output shaft and the second output shaft. Alternatively, the graduated scale is configured for calibrating the displacement of the first output shaft and second output shaft, for instance the relative displacement of, preferably by using predetermined relative displacement.

The present invention also concerns a system for evaluating the relative mobility of a forefoot of a patient, the system comprising

a device according to the present invention;

an orthosis configured for maintaining the forefoot of the patient in position suitable for measuring the relative mobility between the first metatarsal head and the lesser metatarsal heads of a forefoot of a patient;

a positioning module configured for reversibly fastening the device to the orthosis.

In a preferred embodiment, the orthosis is equipped with a sole configured for receiving the foot of the patient. Preferably the sole is molded to the foot of the patient to maintain the feet in a position allowing the forefoot analysis.

The positioning module acts as in interface between the device and the orthosis. The positioning module allow to control the relative position of the orthosis with respect to the device taking the measurements. When the forefoot of the patient is received in the orthosis, the positioning module allows to maintain the feet in a suitable position relative to the device to perform the evaluation of the relative mobility of the forefoot of the patient with the device according to the present invention.

In a preferred embodiment the positioning module comprises reversible fastening means for fastening the positioning module to the orthosis. Preferably, the fastening means also allow to fasten in a reversible manner the positioning module with the device.

Preferably, the reversible fastening means fastening the device to the positioning module are configured for controlling the translation of the device with respect to the positioning module. The translation axis allows to adjust the position of the device along the metatarsal head of the forefoot.

Preferably, the reversible fastening means fastening the orthosis to the positioning module are configured for controlling the translation of the orthosis with respect to the positioning module. The translation axis allows to adjust the position of the device on the metatarsal head of the forefoot.

Advantageously, the positioning module allows the device to be adjusted to each foot along three axes, preferably thee translation axes, meaning in three dimensions (x,y,z).

The positioning module has two main functions: fixing the position of the device relative to the orthosis along two translation axes and secondly allowing the displacement along a third translation axis to bear the device on the forefoot by and exert the axial force on said forefoot.

In other words, firstly, the positioning module allows to immobilize the position of the orthosis and device along two perpendicular translation axes :

- a first translation axis extending along the foot sole once the foot is maintained in the orthosis, and

- a second translation axis perpendicular to said first translation axis to position the device opposite (i.e. under) the metatarsal heads of the forefoot.

Secondly, the device remains displaceable along a third translation axis extending along the sagittal axis to position the first output shaft and the second output shaft respectively opposite the first metatarsal head M1 and the lesser metatarsal heads M2-M5 to exert the axial force on the metatarsal heads and measure the relative displacement.

Preferably, the reversible fastening means comprises a pin and a slider arranged for receiving said pin, the pin being arranged sliding in the slider in a translation motion. Alternatively, the fastening means can comprise knurled screw, worm gear and pinion.

In a preferred embodiment, the device comprises a connection module for connecting said device with an external module. The connection module allows a wireless transfer of the measurements taken by the device, for instance the applied force or the displacement measurements. For instance, the connection module comprises a wireless module for connection in a wireless manner the device with an external module. For instance, the external module is a computation unit configured for processing the measurements acquired by the measuring module to evaluate the relative mobility as a function of the measured axial force with respect to the measured relative displacement.

Alternatively, the external module is a display module to display the data processed by the computation module embedded in the device, meaning in the housing of the device, to evaluate the relative mobility as a function of the measured axial force with respect to the measured relative displacement.

The present invention also relates to a method, preferably computer implemented method, for computing a relative mobility score of a forefoot of a patient, the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and

yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);

iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

iv) computing the relative mobility score RM as

$$RM = Ymax - Ymin.$$

Advantageously, the relative mobility score RM is a parameter allowing to determine the mobility of the first ray which plays an important role in various pathologies of the foot (e.g. Hallux valgus). An indication of the first ray mobility assists the practitioner to make the most appropriate therapeutic decision for the patient (e.g. the type of surgical intervention in case of an operation of the forefoot)

Preferably,

Xmin is comprised between about 0 N and about 30 N, preferably between about 0 N and about 20 N, more preferably between about 5 N and 20 N, more preferably between about 5 N and 15 N, more preferably between about 5 N and 10 Nm
and/or

Xmax is comprised between about 60 N and about 120 N, preferably between about 80 N and about 110 N, more preferably between about 80 N and 100 N, more preferably between about 80 N and 90 N

Preferably, Xmin is between about 5N and about 10N to remove or limit the background noise such as involuntary motion that might occur below or equal to about 5 N.

Preferably, Xmax is comprised between about 90 N and 100 N to ensure to have reached an equilibrium zone (zone 3 - see figure 12).
Preferably, Xmax depends on the position of the patient. For instance, Xmax can be higher than 120 N when the patient is in a standing position.
Preferably, Ymin and Ymax vary and depend on the forefoot of the patient.
The invention further relates to a method, preferably computer implemented method, for computing a differential stiffness score of a forefoot of a patient, the method comprising

i) providing a set of data (xi,yi) where

xi represents a measured applied force along the sagittal axis and equally distributed on the first metatarsal head M1 of the forefoot and on the lesser metatarsal heads M2-M5 of the forefoot, and

yi represents a relative displacement of the first metatarsal head M1 relative to the lesser metatarsal heads M2-M5 along said sagittal axis upon application of the corresponding measured xi applied force,

ii) representing the relative displacement of the first metatarsal head M1 relative to the lesser metatarsal heads M2-M5 (yi) as a function of the measured applied force (xi) for each (xi,yi);

iii) determining Xmin and Xmax as respectively the minimum measured force applied and the maximum measured force applied, and
determining Ymin and Ymax as respectively the minimum measured relative displacement for Xmin and the maximum measured relative displacement at Xmax,

iv) calculating the local derivatives at any points of the curve representing the relative displacement as a function of the force applied between Xmin, Xmax and Ymin, Ymax.

v) computing the differential stiffness score as the maximum value of the calculated local derivatives.

Advantageously, the differential stiffness score is a parameter allowing to determine an indication of the elastic properties of the first ray of the foot.
In another aspect, the invention relates to a method for evaluating the relative mobility of a forefoot of a patient, the method comprising

i) applying an axial force on the forefoot of the patient along the sagittal axis, said axial force being equally distributed between the first metatarsal head M1 relative to the lesser metatarsal heads M2-M5,

ii) measuring a relative displacement of the first metatarsal head M1 relative to the lesser metatarsal heads M2-M5 while applying said axial force;

iii) correlating the measured displacements with the applied axial forces to determine the evolution of the relative displacement of the first metatarsal head M1 relative to the lesser metatarsal heads M2-M5 as a function of the force applied;

The particular advantages of the methods are similar to the ones of the device of the invention and will thus not be repeated here.
As used herein, the word "means" (singular or plural) preceded or followed by a function can be replaced by the word "unit" or " module". For instance "fastening means" can be replaced by "fastening unit" or "fastening module".

As used herein, the term "about" applies to numeric values or ranges of numeric values and refers to a range of numbers that one of skill in the art would consider equivalent to the recited values, i.e. plus or minus ten percent. For example, "about 10 cm" refers to 10 cm +/- 10%, i.e. 9 cm to 11 cm.

The embodiments describe for the device also apply to the methods or the system according to the present invention mutatis mutandis.

## Brief description of the drawings

Further particular advantages and features of the invention will become more apparent from the following non-limitative description of at least one embodiment of the invention which will refer to the accompanying drawings, wherein

- Figures 1 to 7 represent the device and the system according to the present invention;
- Figures 8 and 9 represent the positioning module of the system according to the present invention;
- Figures 10 and 11 represent the equal force distribution on the applied force on the first output shaft and on the second output shaft; figure 10 : F : applied force (N), F1 = F2 : the applied force (F) equally distributed in two forces; k1 and k2 : Stiffness coefficients exerted on the metatarsal supports by the first ray and rays two to five(M1 and M2M5))
- Figure 12 and 13 illustrate the relative mobility score computed by the method according to the present invention;

## Detailed description of the invention

The present detailed description is intended to illustrate the invention in a non-limitative manner since any feature of an embodiment may be combined with any other feature of a different embodiment in an advantageous manner.

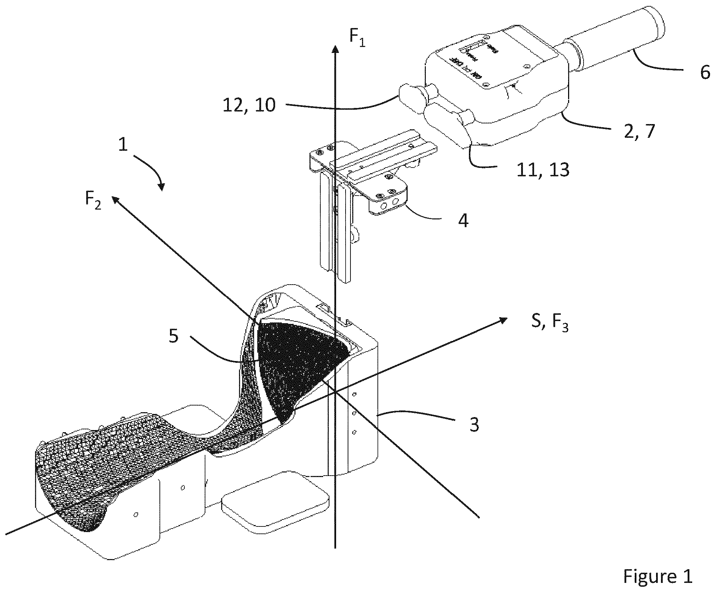

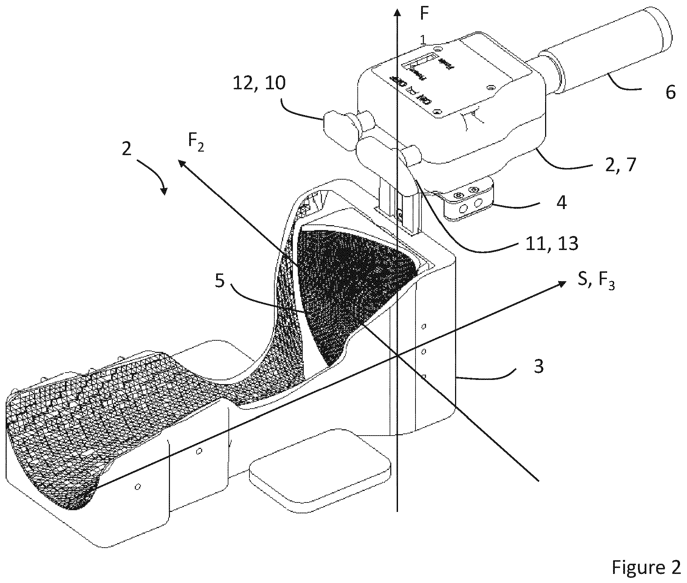

Figures 1 to 4 represent a general overview of an embodiment of the system 1 for evaluating the relative mobility of a forefoot of a patient according to the present invention. However, the invention is not limited to the example shown in the figures and detailed below.

The system 1 comprises a device 2 mounted on an orthosis 3 via a positioning module 4. The device 2, the orthosis 3 and the positioning module 4 are attachable and detachable one another, in other words reversibly attached, the system 1 is modular. This facilitates the transport and/or the storing of the system 1 since the different components can be disassembled before and store in a container suitable for the disassembled system (but unsuitable for the assembled system 1).

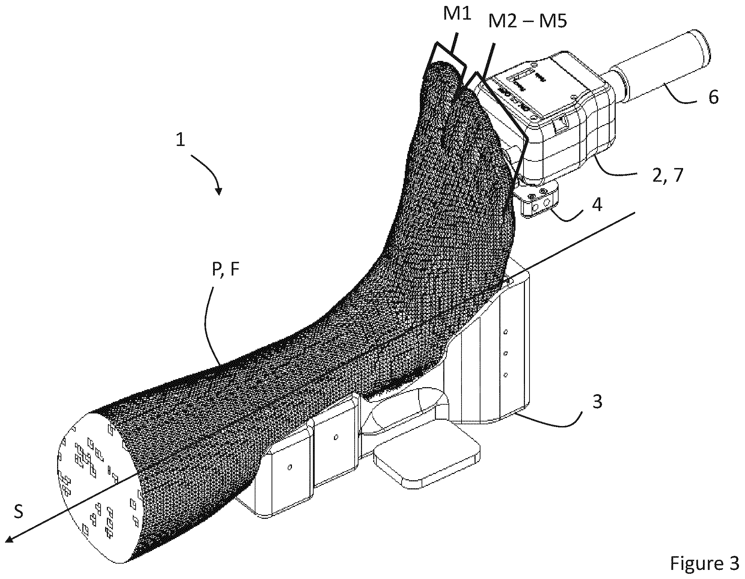

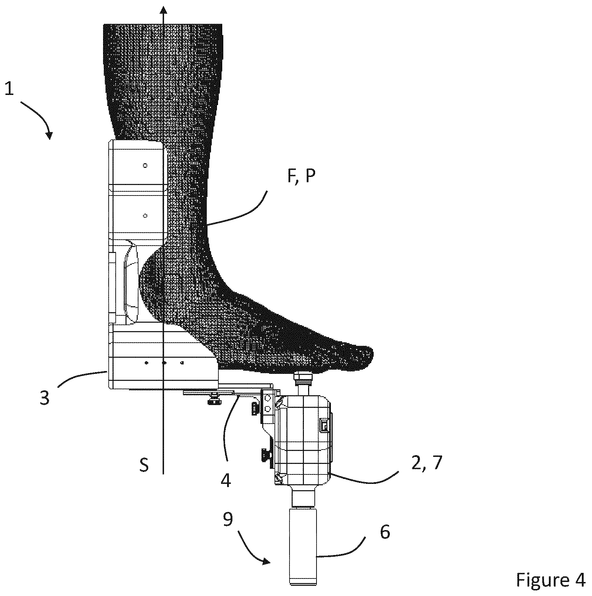

As shown in figures 3 and 4, the orthosis 3 is configured for receiving the foot F of the patient P. the patient P can be either in a lying position as shown in figure 3 or in a sanding position as shown in figure 4. In other embodiments (not shown), the patient can be in any positions between the standing position shown in figure 4 and the lying position shown in figure 3.

In the illustrated embodiment, the orthosis 3 is equipped with a sole 5 configured for receiving the foot of the patient. preferably, a right foot sole is mounted when a right foot is received in the orthosis 3 and a left foot sole is mounted when a left foot is mounted in the orthosis. The orthosis can therefore be used with both feet. In the present example, the orthosis 3 is equipped with a right foot sole.

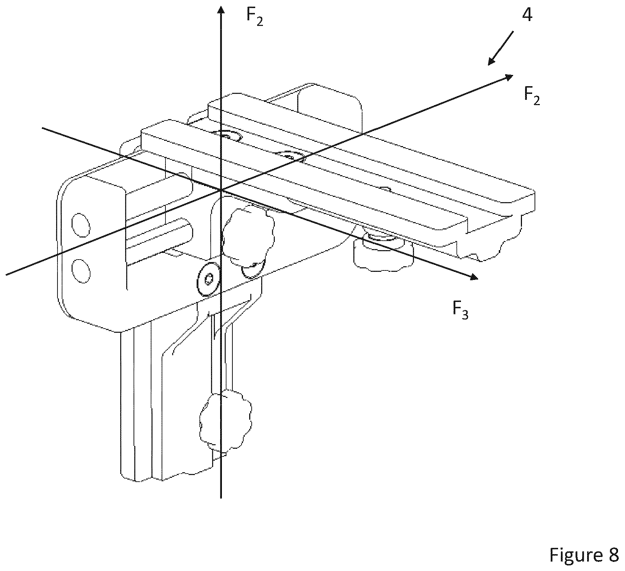

As shown in figures 1,2 and 8,9, in the present example the positioning module allows the device to be adjusted to each foot along three translation axes in three dimensions (x,y,z).

The device, the orthosis and the device are assembled as followed :

- fixing the position of the device relative to the orthosis along two translation axes F1 and F2 ;

- allowing the displacement along a third translation axis F3 to bear the device on the forefoot by and exert the axial force on said forefoot.

In other words, firstly, the positioning module immobilizes the position of the orthosis and device along two perpendicular translation axes :

- a first translation axis F1 extending along the foot sole once the foot is maintained in the orthosis, and

- a second translation axis F2 perpendicular to said first translation axis to position the device opposite (i.e. under) the metatarsal heads of the forefoot.

Secondly, the device remains displaceable along a third translation axis extending F3 along the sagittal axis to position the first output shaft and the second output shaft respectively opposite the first metatarsal head M1 and the lesser metatarsal heads M2-M5 to exert the axial force on the metatarsal heads and measure the relative displacement.

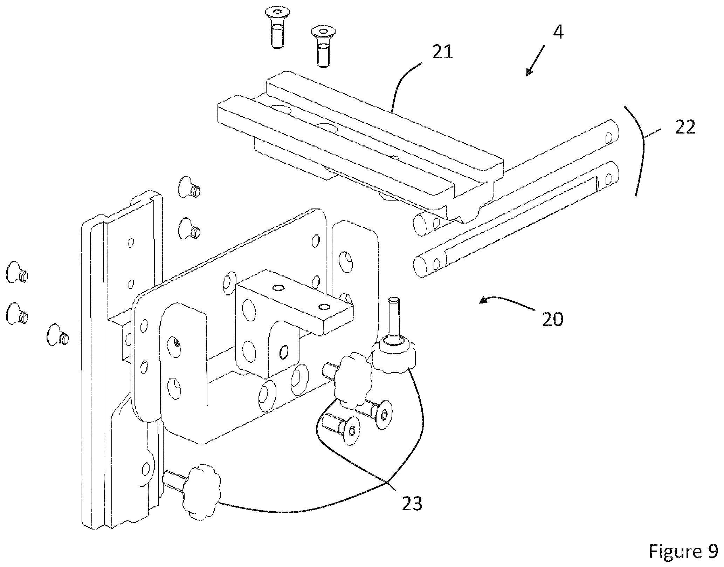

The positioning module 4 comprises fastening means for adjusting the relative position of the device relative to the orthosis 3 and to the forefoot F received in said orthosis 3. As shown in figure 9, in the present example the fastening means comprises a sliding component assembly 20, notably sliding supports 21 sliding along sliding pins 22 and stoppers to lock the positions of said sliding supports 21.

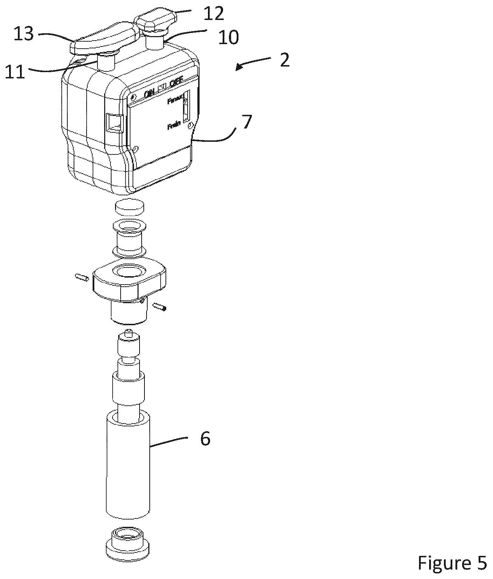

Figures 5 to 7 illustrated in a detailed manner the device 2 according to the present invention. The device 2 comprises a housing 7 hosting the various components of the device 2.

The device 2 further comprises a transmission element 8, and actuator 9, and two outputs shafts namely a first output shaft 10 and a second output shaft 11. The transmission element 8 is configured for equally distributing an axial force provided by the actuator 9 along the sagittal axis S of the patient P to the first output shaft 10 and to the second output shaft 11. The first shaft 10 is configured for bearing against the first metatarsal head M1 of the forefoot whereas the second shaft being configured for bearing against the lesser metatarsal heads M2-M5 of the forefoot. The device further comprises a first metatarsal head M1 tip 12 and a lesser metatarsal heads tip M2-M5 13 reversibly mounted on respectively the first output shaft 10 and the second output shaft 11.

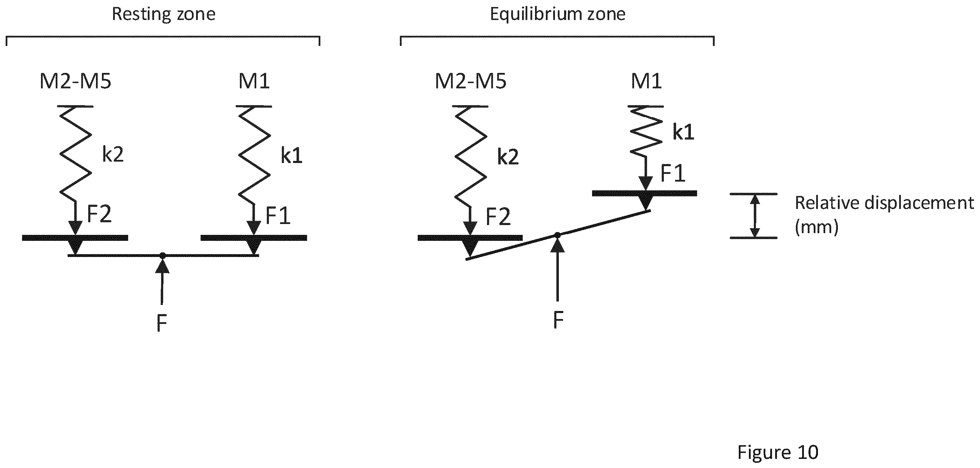

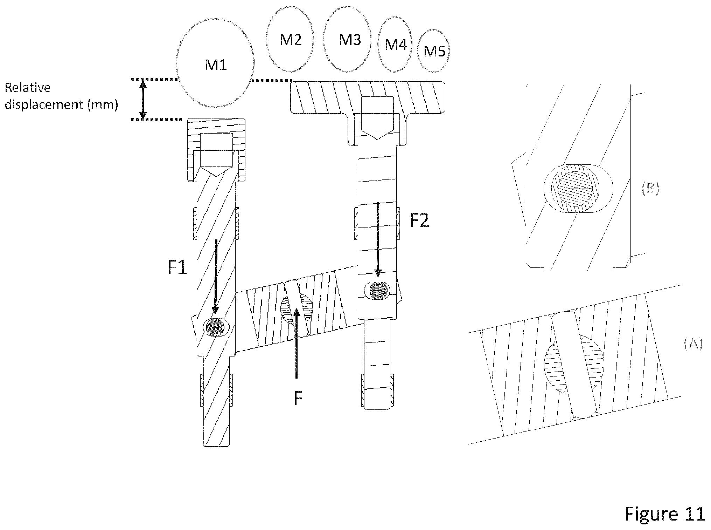

In other words, the force exerted by the actuator 9 is transmitted to the first output shaft 10 and to the second output shaft 11 via the transmission element 8. The transmission element 8 is configured for controlling the force exerted by the actuator 9 to ensure an equal distribution of said force between the first output shaft 10 and the second output shaft 11. This is illustrated in figures 10,11. The force applied F from the actuator 9 is transmitted by the transmission element 8 to the first output shaft 10 and to the second output shaft 11. A force F1 from the first output shaft 10 is applied on the first metatarsal head M1 and a force F2 from the second output shaft 11 is applied on the lesser metatarsal heads M2-M5, F1 being equal to F2.

In the present example, the device 2 is configured for being manually actuated and the actuator 9 comprises the hand 6 outside the housing and fixed to the housing 7. A user, handing the hand 6 as actuator 9, provides or exerts the axial force applied on the transmission element 8.

The transmission element 8 can be any module or components assembly configured for equally distributing the axial force to two output shafts or at least two output shafts. In the present example, the transmission element 8 comprises a pivot plate 14 pivotable around a pivot point P. The pivot plate acts as a balance or a swing tilting on one end or on the other end around the pivot point depending on the effort applied on the respective ends. The actuator 9 is arranged for exerting the axial force on the pivot point P of the pivot plate P.

The first output shaft 10 is mounted on a first end of the pivot plate 14, and the second output shaft 11 is mounted on the opposed end of the pivot plate 14.

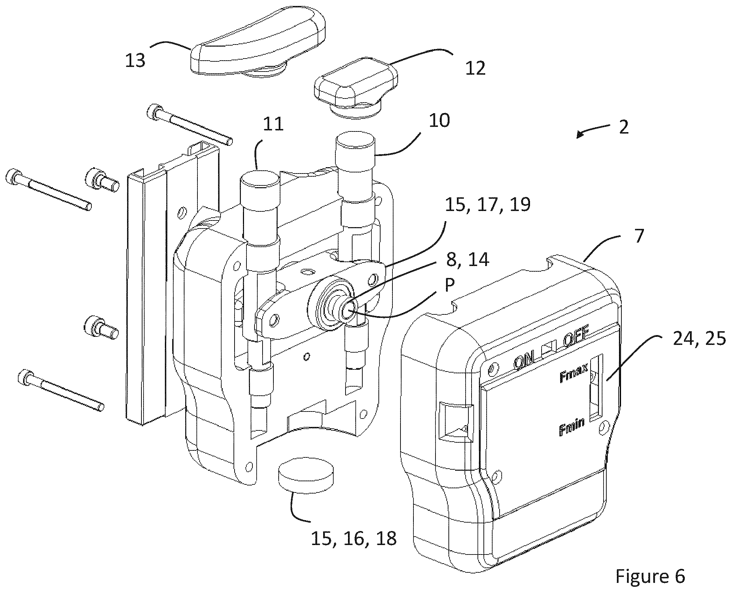

More precisely, according to figure 6, the housing 7 comprises two parallel longitudinal lodging wherein each output shaft 10, 11 is mobile in translation, and a central cavity wherein the pivot plate 14 is located. The central cavity and the two parallel longitudinal lodging are communicating. Each longitudinal lodgings are coming out at one extremity of the housing to let the output shafts 10, 11 protrude out from the housing 7.

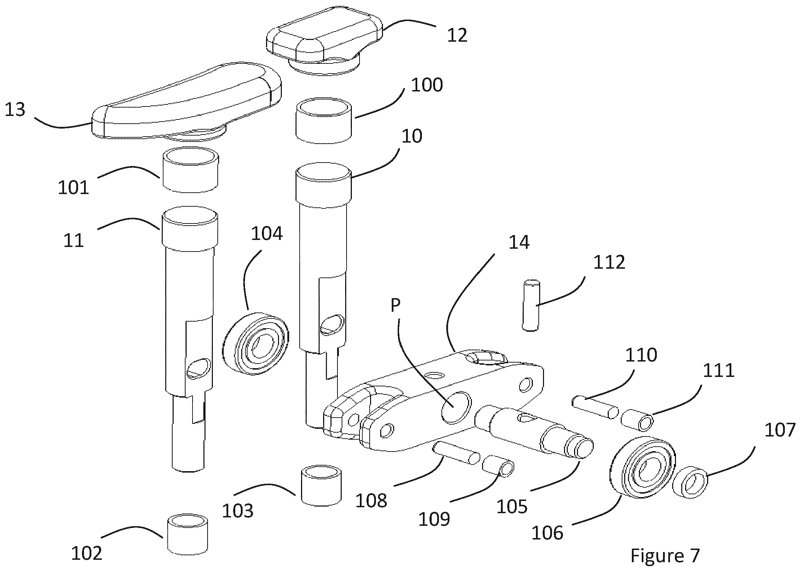

According to figure 7, at each end of a longitudinal lodging are located a plain bearing 100, 101, 102, 103 interdependent to the housing 7, wherein an output shaft 10,11 can freely slide.

The pivot plate 14 comprises a central hole centred with the pivot point P, in which a transverse axis 105 is inserted. At each end of the transverse axis 105, a lateral bearing 104, 106 is located interdependent to the housing 7.

Each lateral bearing 104, 105 are interdependent to the housing 7. More precisely, the transmission of the force between the transverse axis 105 and the pivot plate 14 is carried out by the two lateral bearings 104, 105, fixed on the one hand on the transverse axis 105 and on the other hand on the housing 7.

The transverse axis 105 is fixed to the pivot plate 14 by an orthogonal pin 112, with adapted hole thereof.

The two lateral bearings 104, 105 allow to rotate the transverse axis 105 and the pivot plate 14 relative to the housing 7.

At one end of the transverse axis 105 is inserted a magnet 107. The magnet 107 is magnetized diametrically. The magnet 107 is located in front a magnetic angular sensor 19 fixed to the housing 7 and positioned axially with the magnet 107. The magnetic angular sensor 19 is intended to measure the relative displacement, as explained further in the description.

Each output shaft 10, 11 comprises an oblong aperture, and each end of the pivot plate 14 comprises a circular hole in which a pin 108, 110 via a transversal plain bearing 109, 111 is inserted.

More precisely, each end of the pivot plate 14 comprises a transversal recess to receive one output shaft 10, 11. The output shafts 10, 11 comprises planes within its circular profile to fit in the transversal recesses of the pivot plate 14.

The two transversal plain bearings 109, 111 allow each pins 108, 110 integral with the pivot plate 14 to move horizontally inside the oblong holes in order to slide the output shafts 10, 11 vertically.

Each circular hole at each end of the pivot plate 14 is equidistant to the pivot point P.

A pin 108, 110 is thus inserted through a circular hole and an oblong aperture.

The oblong aperture located on each output shaft 10, 11 allows the conversion of the rotary movement of the pivot plate 14 along the pivot point P into a linear movement of the output shafts 10, 11.

Without any force exerted on the housing 7, the two output shafts 10, 11 are free in motion within the housing 7, interconnected by the pivot plate 14.

According to figure 6, the housing 7 further comprises an exterior recess, at its lower ends, wherein the force sensor 16 is embedded. The exterior recess is opposite to the longitudinal lodgings opening, more precisely opposes according to parallel axis to the two longitudinal lodgings openings, equidistant to the two output shaft 10, 11.

The actuator 9 is aligned according to parallel axis with the two output shafts 10,11. A force exerted on the force sensor 16 by the actuator 6, is transmitted axially to the housing 7, and thus transmitted to the bearings 104, 106, the pivot plate 14 and equally distributed to the two output shafts 10, 11.

As the first metatarsal head M1 tip 12 and the lesser metatarsal heads tip M2-M5 13 are intended to be pressed against a foot of a patient, a force applied by the actuator 9 to the force sensor 16 and to the all housing 7 thereof, allow the pivot plate 14 to transmit an equal force to the first output shaft 10 and to the second output shaft 11, through the transverse axis 105.

More precisely this mechanism allows to measure a relative displacement between the two tips M1 12, M2M5 13 produced by the pivot plate 14 which distributes the force F applied by the actuator 9 equitably into two equal forces F1 and F2 (F=F1+F2;F1=F2=F/2) by the equal length between the pivot point P of the pivot plate 14 and each output shafts 10, 11.

The relative displacement is concretely measured through the angulation of the pivot plate 14, through the rotation of the transverse axis 105 which rotate the magnet 107.

Thus, the relative displacement, i.e., the relative mobility score RM, corresponding to the vertical displacement between the tip M1 12 and the tip M2M5 13, is carried out by the magnetic sensor fixed on the housing 7 and positioned in the axis of the magnet 107.

The magnetic sensor detects the variations of the magnetic field angles along the X and Y axis. The angle of rotation of the pivot plate 14 is calculated according to the following formula: angle = arctangent (Bx/By).

Bx stands for the magnetic field detected by the magnetic sensor along the X axis, whereas By stands for the magnetic field detected by the magnetic sensor along the Y axis. The axes X and Y are reference axes imposed by the magnetic sensor and they depend on the chosen placement of the magnetic sensor.

Knowing the length of the pivot plate 14, the relative displacement can be found by trigonometry according to the following equation: RM = sine(angle) * length.

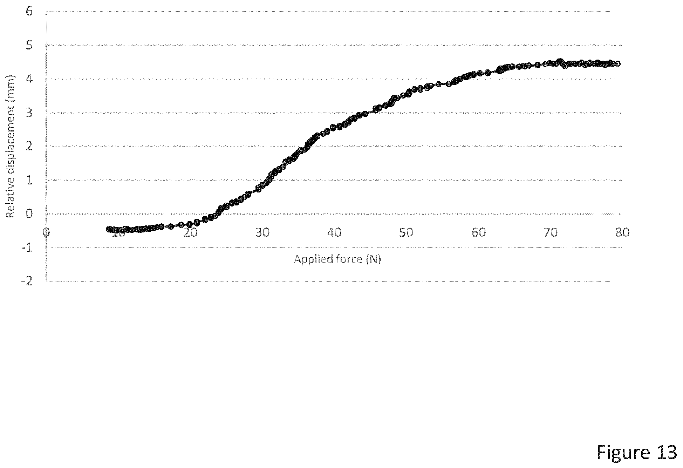

According to figure 13, the measurement curve is obtained by simultaneously displaying the exerted force "F", measured by the force sensor 16 as well as the measurement of relative mobility score RM obtained by measuring the orientation of the pivot plate 14 by the combination of the magnetic sensor 19 and the magnet 107.

As shown in figures 10,11, when the axial force is exerted on the pivot plate 14 and equally distributed to the first output shaft 10 and to the second output shaft 11, the pivot plate 14 is configured for adopting a first position or a second position,

- a first position P1 corresponding resting zone (zone 1 in figure 12- ) where the effort exerted by the first output shaft 10 on the pivot plate 14 is equal to the effort applied by the second output shaft 11 on the pivot plate 14, and

- a second position P2 corresponding to an equilibrium zone (zone 3 in figure 12) where the effort exerted by the first input shaft 10 on the pivot plate 14 is different from the effort applied by the second input shaft 11 on the pivot plate 14; either F2 is superior to F1 (as shown in figures 10,11) or F1 is superior to F2 (not shown).

When the pivot plate is in position P2, a relative displacement between the first metatarsal head M1 and the lesser metatarsal heads can be measured by a measuring module 15.

The measuring module 15 comprises

a force sensor 16 configured for measuring the axial force exerted by the actuator 9, and

a displacement sensor 17 configured for measuring the relative displacement of the first metatarsal head M1 relative to the lesser metatarsal heads M2-M5 along said sagittal axis when beard respectively against the first output shaft 10 and on the second output shaft 11.

In the present embodiment, the force sensor 16 is a compressible cell load 18. The displacement sensor is a magnetic angular sensor 19 (inside the housing 7-not shown in figures).

As shown in figure 6, the device further comprises a feedback module 24 to help the practitioner to evaluate and measure the force applied on the forefoot. In the present example, the feedback module is a visual display meaning a graduated scale 25 between Fmin and Fmax to evaluate the axial force applied by the partitioner on the actuator 9.

The measuring module 15 is configured for correlating the displacement measurements made by the magnetic angular sensor in this example with the measured axial forces made by the compression cell load in this example to evaluate the relative mobility of the forefoot of the patient. In this example, the force sensor 16 and the relative displacement sensor 17 operate simultaneously so that each measured axial force (xi) is associated with a measured relative displacement (yi). Therefore, it is possible to determine a relative mobility score RM corresponding to the displacement of the first metatarsal head M1 relative to the lesser metatarsal heads M2-M5 as a function of the axial force applied. This is shown in figures 12,13.

Figure 12 represents a theorical scheme or curve of the displacement of the first metatarsal head M1 relative to the lesser metatarsal heads M2-M5 (on the y axis) as a function of the axial force applied (on the x axis).

The theorical curve comprises three zones or areas:

- zone 1: resting zone corresponding to a plateau, during which the foot remains at a resting position. In other words, the applied force is incapable of causing a displacement between the first metatarsal head M1 and the lesser metatarsal heads M2-M5;

- zone 2: elastic zone corresponding to a dynamic displacement between the first metatarsal head M1 and the lesser metatarsal heads M2-M5 as a function of the applied force where the relative displacement increases with the applied force.

- zone 3 : equilibrium zone corresponding to a portion where the curve (relative displacement Yi as a function of the applied force xi) depicts a second plateau, during which the maximum relative displacement has been obtained. No additional displacement will be detected when the applied force increases.

In the present case shown in figure 12, Fmin corresponds to X1 and Fmax corresponds to X3 while Ymin corresponds to Y1 and Y3 corresponds to Ymax.

Figure 13 is an example of a calculate RM for a patient :

$$F\ min = 10N$$

$$Fmax = 80N$$

$$Ymin = -0.48mm$$

$$Ymax = 4.46mm$$

$$RM = 4.46 - (-0.048) = 4.94mm$$

**Claims**

1. Device (2) for evaluating the relative mobility of a forefoot (F) of a patient (P), the device (2) comprising an actuator (9), a force sensor (16), a housing (7), a transmission element (8) and two output shafts namely a first output shaft (10) and a second output shaft (11),

   the first output shaft (10) being configured for bearing against the first metatarsal head M1 of the forefoot whereas the second output shaft (11) being configured for bearing against the lesser metatarsal heads M2-M5 of the forefoot, the force sensor (16) is able to measure an axial force exerted by the actuator (9) to the two output shafts (10, 11) bearing to the forefoot, **characterized in that** the transmission element (8) comprises a pivot plate (14) connecting the housing (14) to the two output shafts (10, 11), the pivot plate (14) is able to equally distributes the axial force provided by the actuator (9) via the housing (7), to the first output shaft (10) and to the second output shaft (11),
and wherein the device (2) comprises a displacement sensor (17) able to measure the angulation of the pivot plate (14).

2. Device (2) according to claim 1, wherein the force sensor (16) and the displacement sensor (17) simultaneously measure the force applied and the angulation of the pivot plate (14).

3. Device (2) according to claim 1 or 2, wherein the pivot plate (14) is connected to the housing (7) by a transverse axis (105) and bearings (104, 106).

4. Device (2) according to claim 1 to 3, wherein the first output shaft (10) is mounted on a first end of the pivot plate (14), and the second output shaft (11) is mounted on the opposed end of the pivot plate (14).

5. Device (2) according to any one of the preceding claims, wherein each output shafts (10, 11) comprises an oblong aperture, and each end of the pivot plate (14) comprises a circular hole in which a pin (108, 110) is inserted through the circular hole and the oblong aperture.

6. Device (2) according to any one of the preceding claims, wherein the device (2) comprises plain bearings (100-103) interdependent to the housing (7), wherein the output shafts (10,11) can freely slide.

7. Device (2) according to any one of the preceding claims, wherein the housing (7) comprises an exterior recess, at its lower ends, wherein the force sensor (16) is embedded.

8. Device (2) according to any one of the preceding claims, wherein the device (2) further comprises a computation unit configured for processing the measurements acquired by the force sensor (16) and the displacement sensor (17).

9. Device (2) according to any one of the preceding claims, wherein the device (2) further comprises a first metatarsal head M1 tip (12) configured for being mounted on the first output shaft (10) to be beard against the first metatarsal head M1, and a lesser metatarsal heads M2-M5 tip (13) configured for being mounted on the second output shaft (11) to be beard against the lesser metatarsal heads M2-M5.

10. Device (2) according to the preceding claim, wherein the first metatarsal head M1 tip (12) and the lesser metatarsal heads tip M2-M5 (13) are reversibly mounted on respectively the first output shaft (10) and the second output shaft (11), and wherein the first metatarsal head M1 tip (12) and the lesser metatarsal heads tip M2-M5 (13) are both compatible with the first output shaft (10) and the second output shaft (11).

11. Device (2) according to any one of claims 1 to 8, wherein the transmission module (8) is chosen among a gear transmission, a pivot plate (14) pivotable around a pivot point (P), preferably a pivot plate (14) pivotable around a pivot point (P).

12. Device (2) according to any one of the preceding claims, wherein the displacement sensor (17) is chosen among a magnetic angular sensor (19), an optical encoder, a mechanical encoder, preferably a magnetic angular sensor (19).

13. Device (2) according to any one of claims 3 to 9, wherein the transverse axis (105) comprises at one of its ends a magnet (107) located in front to a magnetic angular sensor (19).

14. Device (2) according to any one of the preceding claims, wherein the force sensor (16) is chosen among cell load, compression cell load (18), spring dynamometer.

15. System (1) for evaluating the relative mobility of a forefoot (F) of a patient (P), the system (1) comprising

 - a device (2) according to any one of the preceding claims
 - an orthosis (3) configured for maintaining the forefoot of the patient in position suitable for measuring the relative mobility between the first metatarsal head and the lesser metatarsal heads of a forefoot of a patient;
 - a positioning module (4) configured for reversibly fastening the device (2) to the orthosis (3).

**Patentansprüche**

1. Vorrichtung (2) zum Beurteilen der relativen Beweglichkeit eines Vorfußes (F) eines Patienten (P), wobei die Vorrichtung (2) einen Aktor (9), einen Kraftsensor (16), ein Gehäuse (7), ein Übertragungselement (8) und zwei Ausgangswellen, nämlich eine erste Ausgangswelle (10) und eine zweite Ausgangswelle (11), umfasst,

 die erste Ausgangswelle (10) dazu ausgelegt ist, gegen den ersten Mittelfußknochenkopf M1 des Vorfußes zu drücken, während die zweite Ausgangswelle (11) dazu ausgelegt ist, gegen die kleineren Mittelfußknochenköpfe M2-M5 des Vorfußes zu drücken,
 der Kraftsensor (16) zum Messen einer axialen Kraft in der Lage ist, die von dem Aktor (9) auf die beiden Ausgangswellen (10, 11) ausgeübt wird, die gegen den Vorfuß drücken,
 **dadurch gekennzeichnet, dass** das Übertragungselement (8) eine Drehplatte (14) umfasst, die das Gehäuse (14) mit den beiden Ausgangswellen (10, 11) verbindet, wobei die Drehplatte (14) in der Lage ist, die von dem Aktor (9) bereitgestellte Axialkraft über das Gehäuse (7) gleichmäßig auf die erste Ausgangswelle (10) und auf die zweite Ausgangswelle (11) zu verteilen,
 und wobei die Vorrichtung (2) einen Verschiebungssensor (17) umfasst, der zum Messen der Abwinkelung der Drehplatte (14) in der Lage ist.

2. Vorrichtung (2) nach Anspruch 1, wobei der Kraftsensor (16) und der Verschiebungssensor (17) gleichzeitig die angewendete Kraft und die Abwinkelung der Drehplatte (14) messen.

3. Vorrichtung (2) nach Anspruch 1 oder 2, wobei die Drehplatte (14) durch eine Querachse (105) und Lager (104, 106) mit dem Gehäuse (7) verbunden ist.

4. Vorrichtung (2) nach Anspruch 1 bis 3, wobei die erste Ausgangswelle (10) an einem ersten Ende der Drehplatte (14) montiert ist und die zweite Ausgangswelle (11) an dem gegenüberliegenden Ende der Drehplatte (14) montiert ist.

5. Vorrichtung (2) nach einem der vorhergehenden Ansprüche, wobei jede Ausgangswelle (10, 11) eine längliche Öffnung umfasst und jedes Ende der Drehplatte (14) ein kreisförmiges Loch umfasst, in das ein Stift (108, 110) durch das kreisförmige Loch und die längliche Öffnung eingeführt wird.

6. Vorrichtung (2) nach einem der vorhergehenden Ansprüche, wobei die Vorrichtung (2) Gleitlager (100-103) umfasst, die mit dem Gehäuse (7) verbunden sind, wobei die Ausgangswellen (10, 11) frei gleiten können.

7. Vorrichtung (2) nach einem der vorhergehenden Ansprüche, wobei das Gehäuse (7) an seinen unten Enden eine äußere Aussparung umfasst, in die der Kraftsensor (16) eingebettet ist.

8. Vorrichtung (2) nach einem der vorhergehenden Ansprüche, wobei die Vorrichtung (2) ferner eine Recheneinheit umfasst, die zum Verarbeiten der von dem Kraftsensor (16) und dem Verschiebungssensor (17) erfassten Messungen ausgelegt ist.

**9.** Vorrichtung (2) nach einem der vorhergehenden Ansprüche, wobei die Vorrichtung (2) eine erste Spitze (12) für den ersten Mittelfußknochenkopf M1, die dazu ausgelegt ist, auf der ersten Ausgangswelle (10) montiert zu werden, um gegen den ersten Mittelfußknochenkopf M1 gedrückt zu werden, und eine Spitze (13) für die kleineren Mittelfußknochenköpfe M2-M5 umfasst, die dazu ausgelegt ist, auf der zweiten Ausgangswelle (11) montiert zu werden, um gegen die kleineren Mittelfußknochenköpfe M2-M5 gedrückt zu werden.

**10.** Vorrichtung (2) nach dem vorhergehenden Anspruch, wobei die Spitze (12) für den ersten Mittelfußknochenkopf M1 und die Spitze (13) für die kleineren Mittelfußknochenköpfe M2-M5 reversibel auf der ersten Ausgangswelle (10) bzw. der zweiten Ausgangswelle (11) montiert sind und wobei die Spitze (12) für den ersten Mittelfußknochenkopf M1 und die Spitze (13) für die kleineren Mittelfußknochenköpfe M2-M5 beide mit der ersten Ausgangswelle (10) und der zweiten Ausgangswelle (11) kompatibel sind.

**11.** Vorrichtung (2) nach einem der Ansprüche 1 bis 8, wobei das Übertragungsmodul (8) aus einem Zahnradgetriebe und einer um einen Drehpunkt (P) drehbaren Drehplatte (14), vorzugsweise einer um einen Drehpunkt (P) drehbaren Drehplatte (14), ausgewählt ist.

**12.** Vorrichtung (2) nach dem vorhergehenden Anspruch, wobei der Verschiebungssensor (17) aus einem magnetischen Winkelsensor (19), einem optischen Encoder und einem mechanischen Encoder, vorzugsweise einem magnetischen Winkelsensor (19), ausgewählt ist.

**13.** Vorrichtung (2) nach einem der Ansprüche 3 bis 9, wobei die Querachse (105) an einem ihrer Enden einen Magneten (107) umfasst, der sich vor einem magnetischen Winkelsensor (19) befindet.

**14.** Vorrichtung (2) nach dem vorhergehenden Anspruch, wobei der Kraftsensor (16) aus einer Kraftmessdose, einem Druckmessode (18) und einem Federdynamometer ausgewählt ist.

**15.** System (1) zum Beurteilen der relativen Beweglichkeit eines Vorfußes (F) eines Patienten (P), wobei das System (1) Folgendes umfasst:

- eine Vorrichtung (2) nach einem der vorhergehenden Ansprüche,
- eine Orthese (3), die dazu ausgelegt ist, den Vorfuß des Patienten in einer Position zu halten, die zum Messen der relativen Beweglichkeit zwischen dem ersten Mittelfußknochenkopf und den kleineren Mittelfußknochenköpfen eines Vorfußes eines Patienten geeignet ist;
- ein Positionierungsmodul (4), das zum reversiblen Befestigen der Vorrichtung (2) an der Orthese (3) ausgelegt ist.

## Revendications

**1.** Dispositif (2) d'évaluation de la mobilité relative de l'avant-pied (F) d'un patient (P), le dispositif (2) comprenant un actionneur (9), un capteur de force (16), un boîtier (7), un élément de transmission (8) et deux arbres de sortie, un premier arbre de sortie (10) et un second arbre de sortie (11),

le premier arbre de sortie (10) est conçu pour s'appuyer sur la tête métatarsienne M1 de l'avant-pied, tandis que le second arbre de sortie (11) est conçu pour s'appuyer sur les têtes métatarsiennes M2 à M5 de l'avant-pied, le capteur de force (16) est capable de mesurer la force axiale exercée par l'actionneur (9) sur les deux arbres de sortie (10, 11) en appui sur l'avant-pied, **caractérisé en ce que** l'élément de transmission (8) comprend une plaque pivotante (14) reliant le boîtier (14) aux deux arbres de sortie (10, 11), la plaque pivotante (14) répartit uniformément la force axiale fournie par l'actionneur (9) via le boîtier (7), au premier arbre de sortie (10) et au deuxième arbre de sortie (11), et dans lequel le dispositif (2) comprend un capteur de déplacement (17) apte à mesurer l'angulation de la plaque pivotante (14).

**2.** Dispositif (2) selon la revendication 1, dans lequel le capteur de force (16) et le capteur de déplacement (17) mesurent simultanément la force appliquée et l'angulation de la plaque pivotante (14).

**3.** Dispositif (2) selon la revendication 1 ou 2, dans lequel la plaque pivotante (14) est reliée au boîtier (7) par un axe transversal (105) et des roulements (104, 106).

**4.** Dispositif (2) selon la revendication 1 à 3, dans lequel le premier arbre de sortie (10) est monté sur une première extrémité de la plaque pivotante (14), et le deuxième arbre de sortie (11) est monté sur l'extrémité opposée de la plaque pivotante (14).

**5.** Dispositif (2) selon l'une quelconque des revendications précédentes, dans lequel chaque arbre de sortie (10, 11) comprend une ouverture oblongue, et chaque extrémité de la plaque pivotante (14) comprend un trou circulaire dans lequel une broche (108, 110) est insérée à travers le trou circulaire et l'ouverture oblongue.

6. Dispositif (2) selon l'une quelconque des revendications précédentes, dans lequel le dispositif (2) comprend des paliers lisses (100-103) interdépendant du boîtier (7), dans lesquels les arbres de sortie (10, 11) peuvent coulisser librement.

7. Dispositif (2) selon l'une quelconque des revendications précédentes, dans lequel le boîtier (7) comprend un évidement extérieur, à ses extrémités inférieures, dans lequel est encastré le capteur de force (16).

8. Dispositif (2) selon l'une quelconque des revendications précédentes, dans lequel le dispositif (2) comprend en outre une unité de calcul configurée pour traiter les mesures acquises par le capteur de force (16) et le capteur de déplacement (17).

9. Dispositif (2) selon l'une quelconque des revendications précédentes, dans lequel le dispositif (2) comprend en outre une première pointe (12) de tête métatarsienne M1 configurée pour être montée sur le premier arbre de sortie (10) pour être portée contre la première tête métatarsienne M1, et une pointe inférieure (13) de têtes métatarsiennes M2-M5 configurée pour être montée sur le deuxième arbre de sortie (11) pour être portée contre les têtes métatarsiennes inférieures M2-M5.

10. Dispositif (2) selon la revendication précédente, dans lequel la première pointe (12) de tête métatarsienne M1 et la pointe inférieure (13) de têtes métatarsiennes M2-M5 sont montées de manière réversible sur respectivement le premier arbre de sortie (10) et le deuxième arbre de sortie (11), et dans lequel la première pointe (12) de tête métatarsienne M1 et la pointe inférieure (13) de têtes métatarsiennes M2-M5 sont toutes deux compatibles avec le premier arbre de sortie (10) et le deuxième arbre de sortie (11).

11. Dispositif (2) selon l'une quelconque des revendications 1 à 8, dans lequel l'élément de transmission (8) est choisi parmi une transmission à engrenages, une plaque pivotante (14) pivotante autour d'un point de pivot (P), de préférence une plaque pivotante (14) pivotante autour d'un point de pivot (P).

12. Dispositif (2) selon l'une quelconque des revendications précédentes, dans lequel le capteur de déplacement (17) est choisi parmi un capteur angulaire magnétique (19), un codeur optique, un codeur mécanique, de préférence un capteur angulaire magnétique (19).

13. Dispositif (2) selon l'une quelconque des revendications 3 à 9, dans lequel l'axe transversal (105) comporte à l'une de ses extrémités un aimant (107) situé devant un capteur angulaire magnétique (19).

14. Dispositif (2) selon l'une quelconque des revendications précédentes, dans lequel le capteur de force (16) est choisi parmi un capteur électro-magnétique, une capteur de compression (18), un dynamomètre à ressort.

15. Système (1) d'évaluation de la mobilité relative d'un avant-pied (F) d'un patient (P), le système (1) comprenant

   - un dispositif (2) selon l'une quelconque des revendications précédentes
   - une orthèse (3) configurée pour maintenir l'avant-pied du patient dans une position appropriée pour mesurer la mobilité relative entre la première tête métatarsienne et les petites têtes métatarsiennes d'un avant-pied d'un patient ;
   - un module de positionnement (4) configuré pour fixer de manière réversible le dispositif (2) à l'orthèse (3).

Figure 1

Figure 2

Figure 3

Figure 4

Figure 5

Figure 6

Figure 7

Figure 8

Figure 9

Figure 10

Figure 11

Figure 12

Figure 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2017171660 A